(19) Europäisches Patentamt
European Patent Office
Office européen des brevets

(11) EP 0 955 045 A1

(12) **EUROPEAN PATENT APPLICATION**
published in accordance with Art. 158(3) EPC

(43) Date of publication:
**10.11.1999 Bulletin 1999/45**

(21) Application number: **97933066.9**

(22) Date of filing: **25.07.1997**

(51) Int. Cl.$^6$: **A61K 31/18**, A61K 9/06, A61K 9/08

(86) International application number:
**PCT/JP97/02629**

(87) International publication number:
**WO 98/05316 (12.02.1998 Gazette 1998/06)**

(84) Designated Contracting States:
**AT BE CH DE DK ES FI FR GB GR IE IT LI LU MC NL PT SE**

(30) Priority: **02.08.1996 JP 22056296**

(71) Applicants:
- **Senju Pharmaceutical Co., Ltd.**
  **Osaka-shi, Osaka 541-0046 (JP)**
- **YAMANOUCHI PHARMACEUTICAL CO., LTD.**
  **Tokyo 103 (JP)**

(72) Inventors:
- **OGAWA, Takahiro**
  **Nishinomiya-shi, Hyogo 662-0865 (JP)**
- **WATANABE, Noriko**
  **Suita-shi, Osaka 564 (JP)**
- **WAKI, Mitsunori**
  **Kobe-shi, Hyogo 651-22 (JP)**

(74) Representative:
**Burford, Anthony Frederick**
**W.H. Beck, Greener & Co.**
**7 Stone Buildings**
**Lincoln's Inn**
**London WC2A 3SZ (GB)**

# (54) DRUGS FOR IMPROVING OCULAR CIRCULATION DISORDERS

(57) An anti-ocular circulatory disturbance composition comprising 5-{1-hydroxy-2-[2-(2-methoxyphenoxy)ethylamino]ethyl}-2-methylbenzenesulfonamide or its acid addition salt as an active ingredient. This composition can be used as a therapeutic and prophylactic drug for diseases caused by ocular circulatory disturbance, such as normal tension glaucoma, pigmentary retinal degeneration, macular regeneration, ischemic optic neuropathy, iridocyclitis, retinal artery occlusion, retinal vein occlusion, diabetic retinopathy, choroidal disease secondary to retinal lesions, and retina-choroidal disease, without a risk for adverse reactions. The composition shows excellent efficacy even by topical administration.

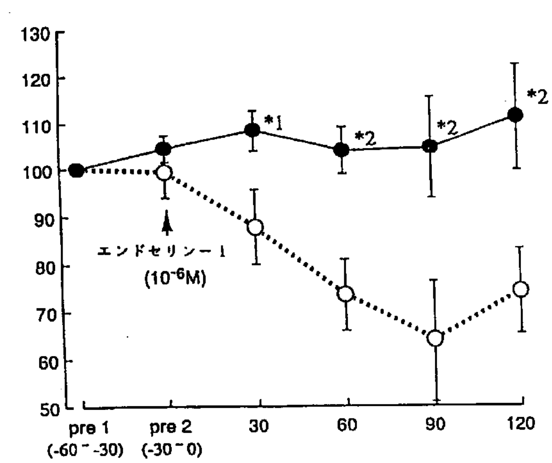


EP 0 955 045 A1

## Description

Technical Field

The present invention relates to an anti-ocular circulatory disturbance composition. More particularly, the invention relates to an anti-ocular circulatory disturbance composition comprising 5-[1-hydroxy-2-[2-(2-methoxyphenoxy)ethyl-amino]ethyl]-2-methylbenzenesulfonamide or an acid addition salt thereof as an active ingredient.

Background Art

In the eye, there are two major systems for blood circulation (hereinafter referred to as ocular circulation), namely the circulation via the ciliary artery and the circulation via the central retinal artery. The ciliary artery communicates with the arteries feeding the choroid, optic nerve head, iris, and ciliary body, and the blood drains through the vortex vein. On the other hand, the central retinal artery pierces the optic nerve into communication with the central retinal vein and partially branches into arterioles in the optic nerve head and further into capillaries. A circulatory disturbance of the ciliary arterial system leads to normal tension glaucoma, pigmentary retinal degeneration, macular degeneration, ischemic optic neuropathy, and iridocyclitis. A circulatory disturbance of the central retinal arterial system results in such diseases as retinal artery occlusion, retinal vein occlusion, diabetic retinopathy, ischemic optic neuropathy, choroidal disease secondary to retinal lesions, and retina-choroidal disease accompanied by systemic disorders. As evident from the above schematic description of ocular circulation, the above-mentioned diseases caused by ocular circulatory disturbance develop when the circulation of blood in the retina, optic nerve head, choroid, iris, or ciliary body is disturbed.

Recent years have seen an increased incidence of normal tension glaucoma (also known as low tension glaucoma). Recent epidemiologic investigations have revealed, with increasing clarity, that normal tension glaucoma is the most prevalent type of glaucoma despite the fact that the patient's intraocular pressure is within the normal range, clearly differentiating itself from high tension glaucoma, which is commonly called glaucoma and etiologically associated with elevation of intraocular pressure. Normal tension glaucoma is generally characterized by (I) an intraocular pressure, inclusive of diurnal rhythm, of not higher than 21 mmHg, (2) open angle, (3) glaucomatous cupping of the optic nerve head and the corresponding visual field defect, (4) absence of intracranial lesions or paranasal sinus disorders which may cause optic atrophy, and (5) no past history of massive hemorrhage and shock [Low tension glaucoma and endothelin (ET-1), Folia Ophthalmologica Japonica, 43, 554-559, 1992 and Low tension glaucoma - Its history and concept, Journal of the Eye, 8, 493-500, 1991].

As the mechanisms for onset of normal tension glaucoma, (1) circulatory disorder of the optic nerve head and (2) mechanical compression have been pointed out, but the real cause remains to be known for certain yet. However, it has been reported that oral administration of a hemodynamic circulation improving agent to patients with normal tension glaucoma resulted in an increased blood flow at the optic nerve head and an improvement of visual field defect in normal tension glaucoma [The Effect of a $Ca^{2+}$ channel blocker on the alteration of visual field in low tension glaucoma, Journal of Japanese Ophthalmological Society, 92, 792-797, 1988). Furthermore, it has been reported that the blood concentration of endothelin-1 (hereinafter sometimes referred to briefly as ET-1), a substance having physiological vasoconstrictive activity, is significantly higher in patients with normal tension glaucoma than in normal subjects [Low-tension glaucoma and endothelin-1 (ET-1), Folia Ophthalmologica Japonica, 43, 554-559, 1992]. It is thought that ET-1 is associated with morbidity of normal tension glaucoma.

It is also documented that intravitreous injection of ET-1 decreases the blood flow on the optic nerve head [The effect of endothelin-1 on intraocular circulation, Journal of Japanese Ophthalmological Society, 97, No. 6, 678-682, 1993]. These findings suggest that as ET-1 decreases the blood flow on the optic nerve head, it induces retina-choroidal circulatory disturbance, thus reducing the optic nerve head blood flow. Therefore, it is considered that improving the ET-1-induced ocular circulatory disturbance can be an effective therapeutic approach to normal tension glaucoma.

Ocular circulatory disturbance is the most frequent lesion found in retina-choroidal diseases. The retina-choroidal disease caused by ocular circulatory disturbance includes retinal artery occlusion, retinal vein occlusion, diabetic retinopathy, pigmentary retinal degeneration, macular degeneration, choroidal disease secondary to retinal lesions, and retina-choroidal disease accompanied by systemic disorders. Although the cause for onset of retinal artery occlusion or retinal vein occlusion is unknown, arterial or venous luminal emphraxis leads to a disorder of retinal circulation and a circulatory disturbance in the optic nerve head. It has also been reported that the blood ET-1 concentration is high in these patients (Journal of Clinical Ophthalmology, 46, 431-434, 1992). It is well known that in diabetic retinopathy, too, the formation of thrombi in the retinal vessels is a cause of retinal circulatory disturbance. Pigmentary retinal degeneration is a bilateral retinal disease which has its onset in the school age with night blindness and is characterized by progressive exacerbation of visual field defects and impaired visual acuity, which may end with loss of sight. This disease is an inherited disease, in which the progressive degeneration of retinal visual cells results in a gradual narrowing of the retina-choroidal blood vessels, which may cause a circulatory disturbance. In macular degeneration, too, a disorder of ocular circulation is said to be involved. As a pharmacotherapy for the above diseases involving ocular circulatory disturbance, oral administration of tocopherol nicotinate (Juvela N: a vitamin E preparation available from Eisai Co. Ltd.) is the current therapy.

The optic nerve disease involving a disorder of the optic nerve includes ischemic optic neuropathy. Isehemic optic neuropathy develops from a circulatory disturbance of optic nerve-feeding vessels. As a disease involving a disturbance of iridociliary circulation, there can be mentioned iridocyclitis.

Meanwhile, there has not been discovered a drug showing satisfactory anti-ocular circulatory disturbance activity without entailing toxic or adverse reactions. The present invention, therefore, has for its object to provide a drug capable of antagonizing circulatory insufficiency of the eye without high toxicological and adverse reaction.

Under the circumstances the inventors of the present invention explored into a variety of compounds in search for a substance that would show satisfactory anti-ocular circulatory disturbance activity without toxicity. As a consequence, the inventors discovered that 5-{1-hydroxy-2-[2-(2-methoxyphenoxy)ethylamino]ethyl}-2-methylbenzenesulfonamide (this compound is hereinafter sometimes referred to as amosulalol and the corresponding hydrochloride as amosulalol hydrochloride; both are collectively referred to as the compound of the invention), which is the active ingredient of the anti-ocular circulatory disturbance composition of the present invention, exhibits an unexpectedly high anti-ocular circulatory disturbance action and, accordingly, have perfected the present invention.

While the compound of the invention is a species of the phenylethanolamine derivative and salt disclosed in JP-A-54-95544, a patent application filed by one of the present applicants, this literature does not disclose any further information other than the statement that the compound of the invention among others has both α-adrenergic and β-adrenergic receptor blocking actions and is useful as an antihypertensive drug or an antianginal drug.

JP-A-55-73610 also discloses the same information as above but no further information.

The present Applicants discovered further that the compound of the invention has an ocular hypotensive action and, based on the finding, applied for a patent for the use of the compound in the treatment of glaucoma (PCT/JP93/00654, WO93/24121).

However, there is no published literature pointing out or alluding to the anti-ocular circulatory disturbance activity of the compound of the invention, and the present inventors should be credited with the discovery of this activity.

The present invention, therefore, is directed to an anti-ocular circulatory disturbance composition comprising 5-{1-hydroxy-2-[2-(2-methoxyphenoxy)-ethylamino]ethyl}-2-methylbenzenesulfonamide of the following formula or an acid addition salt thereof as an active ingredient.

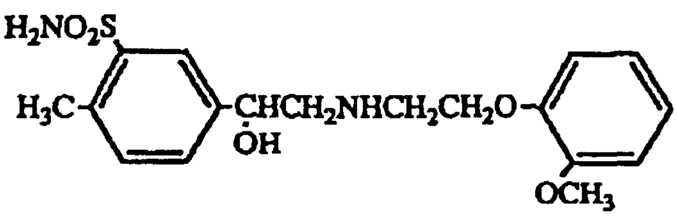

The acid addition salt includes the corresponding salts with inorganic acids such as hydrochloric acid, sulfuric acid, etc. and with organic acids such as maleic acid, tartaric acid, citric acid, etc., although the hydrochloride (i.e. amosulalol hydrochloride) is preferred.

The physiochemical properties and processes for production of amosulalol or its acid addition salt for use as the active ingredient of the anti-ocular circulatory disturbance composition of the present invention are described in inter alia JP-A-54-95544 referred to above.

As will be apparent from the test example presented hereinafter, the anti-ocular circulatory disturbance composition of the present invention has very satisfactory anti-ocular circulatory disturbance activity without toxic or adverse reactions and, therefore, can be used as a safe drug in the prevention and treatment of various diseases caused by ocular circulatory disturbance, such as normal tension glaucoma, retinal artery occlusion, retinal vein occlusion, diabetic retinopathy, pigmentary retinal degeneration, macular degeneration, choroidal disease secondary to retinal lesions, retina-choroidal disease accompanied by systemic disorders, ischemic optic neuropathy, and iridocyclitis, among other diseases.

With regard to the use of amosulalol or its acid addition salt as an anti-ocular circulatory disturbance drug, it is generally administered in admixture with a per se known pharmaceutically acceptable carrier, excipient, and/or diluent in the routine manner, for example in a non-oral dosage form such as an ophthalmic solution, an ophthalmic ointment, or an injection, or in an oral dosage form such as tablets, capsules, or granules. However, it is particularly preferable to use it in the form of an aqueous ophthalmic solution.

Thus, when the anti-ocular circulatory disturbance composition is used in the form of an ophthalmic solution, for instance, it can be supplemented with suitable amounts of the additives which are generally used in topical ophthalmic products, such as the buffer, isotonizing agent, preservative, solubilizer (stabilizer), pH adjusting agent, thickener, chelating agent, and others.

Among the above-mentioned additives, the buffer includes phosphate buffer, borate buffer, citrate buffer, tartrate buffer, acetate buffer, and amino acids. Preferred is a salt of acetic acid such as sodium acetate.

The isotonizing agent includes saccharides such as sorbitol, glucose, mannitol, etc., polyhydric alcohols such as glycerol, polyethylene glycol, propylene glycol, etc., and salts such as sodium chloride, although glycerol is preferred.

The preservative includes benzalkonium chloride, benzethonium chloride, p-hydroxybenzoic acid esters, such as methyl p-hydroxybenzoate, ethyl p-hydroxybenzoate, etc., benzyl alcohol, phenethyl alcohol, sorbic acid or its salt, thimerosal, chlorobutanol, etc., although benzalkonium chloride is preferred.

The solubilizer (stabilizer) includes cyclodextrins and their derivatives, water-soluble macromolecular substances such as polyvinylpyrrolidone, and surfactants, among others, although polyvinylpyrrolidone and cyclodextins are preferred.

The pH adjusting agent includes hydrochloric acid, acetic acid, phosphoric acid, sodium hydroxide, potassium hydroxide, etc., although hydrochloric acid is preferred.

The thickener includes hydroxyethylcellulose, hydroxypropylcellulose, methylcellulose, hydroxypropylmethylcellulose, carboxymethylcellulose and its salt, etc.

The chelating agent includes but is not limited to sodium edetate, sodium citrate, and poly(sodium phosphate).

When the anti-ocular circulatory disturbance composition of the present invention is used in the form of an ophthalmic ointment, the ointment base that can be used includes purified lanolin, white petrolatum, plastibase, liquid paraffin, and poly ethylene glycol, among others.

The anti-ocular circulatory disturbance composition of the present invention can also be used in oral dosage forms such as tablets, capsules, granules, etc. or in injectable forms.

The dosage of the anti-ocular circulatory disturbance composition of the present invention depends on the route of administration, the clinical condition to be treated, the patient's age and body weight, and other factors. However, for administration as, for example, an ophthalmic solution to an adult patient with normal tension glaucoma, the active substance amosulalol or acid addition salt is preferably formulated into an aqueous ophthalmic solution of about 0.001-5.0 w/v % or preferably about 0.05-1.0 w/v % concentration and one to a few drops is administered 2-6 times daily depending on the condition of a patient.

When the anti-ocular circulatory disturbance composition of the present invention is used in the form of an ophthalmic ointment, the active substance is preferably formulated into an ophthalmic ointment of about 0.001-10 w/w % or preferably about 0.05-1.0 w/w % concentration and is administered 2-6 times daily depending on the condition of a patient.

Unless contrary to the object of the invention, the anti-ocular circulatory disturbance composition of the present invention may further contain one or more other anti-ocular circulatory disturbance drugs.

Similarly, unless contrary to the object of the invention, the anti-ocular circulatory composition of the present invention may further contain other pharmacologically active substances having other types of efficacy.

The following examples are intended to describe the present invention in further detail and the following test example is intended to demonstrate the efficacy of the invention. It should be understood that these examples are merely illustrative and by no means limitative of the present invention.

Example 1 Ophthalmic Solution

An aqueous ophthalmic solution was provided according to the following formula.

| | |
|---|---|
| Amosulalol hydrochloride | 0.05 g |
| Mannitol | 5.0 g |
| Sodium (mono)hydrogen phosphate | 0.1 g |
| Methyl p-hydroxybenzoate | 0.02 g |
| Propyl p-hydroxybenzoate | 0.01 g |
| Diluted hydrochloric acid | q. s. (pH 6.0) |

(continued)

| | |
|---|---|
| Sterilized purified water to make 100 ml | |

Example 2 Ophthalmic Solution

An aqueous ophthalmic solution was provided according to the following formula.

| | |
|---|---|
| Amosulalol hydrochloride | 0.1 g |
| Boric acid | 2.0 g |
| Benzalkonium chloride | 0.005 g |
| Sodium hydroxide | q. s. (pH 6.0) |
| Sterilized purified water to make 100 ml | |

Example 3 Ophthalmic Solution

An aqueous ophthalmic solution was provided according to the following formula.

| | |
|---|---|
| Amosulalol hydrochloride | 0.25 g |
| Concentrated glycerin | 2.6 g |
| Sodium acetate | 0.1 g |
| $\alpha$-Cyclodextrin | 0.1 g |
| Methyl p-hydroxybenzoate | 0.02 g |
| Propyl p-hydroxybenzoate | 0.01 g |
| Diluted hydrochloric acid | q. s. (pH 4.5) |
| Sterilized purified water to make 100 ml | |

Example 4 Ophthalmic Solution

An aqueous ophthalmic solution was provided according to the following formula.

| | |
|---|---|
| Amosulalol hydrochloride | 0.5 g |
| Concentrated glycerin | 2.6 g |
| Sodium acetate | 0.1 g |
| Benzalkonium chloride | 0.005 g |
| Diluted hydrochloric acid | q. s. (pH 5.0) |
| Sterilized purified water to make 100 ml. | |

Example 5 Ophthalmic Solution

An aqueous ophthalmic solution was provided according to the following formula.

| | |
|---|---|
| Amosulalol hydrochloride | 0.5 g |
| Concentrated glycerin | 2.6 g |
| Sodium acetate | 0.1 g |
| Polyvinylpyrrolidone | 0.5 g |
| Benzalkonium chloride | 0.005 g |
| Diluted hydrochloric acid | q. s. (pH 5.5) |
| Sterilized purified water to make 100 ml | |

Example 6 Ophthalmic Solution

An aqueous ophthalmic solution was provided according to the following formula.

| | |
|---|---|
| Amosulalol hydrochloride | 1.0 g |
| Concentrated glycerin | 2.6 g |
| Sodium monohydrogen phosphate | 0.1 g |
| Polyvinylpyrrolidone | 1.0 g |
| Sodium edetate | 0.05 g |
| Benzalkonium chloride | 0.005 g |
| Sodium hydroxide | q. s. (pH 4.0) |
| Sterilized purified water to make 100 ml | |

Example 7 Ophthalmic Solution

An aqueous ophthalmic solution was provided according to the following formula.

| | |
|---|---|
| Amosulalol hydrochloride | 0.5 g |
| Concentrated glycerin | 0.1 g |
| Sodium acetate | 2.6 g |
| Polyvinylpyrrolidone | 0.1 g |
| Benzalkonium chloride | 0.005 g |
| Diluted hydrochloric acid | q. s. (pH 5.0) |
| Sterilized purified water to make 100 ml | |

Example 8 Ophthalmic Ointment

An ophthalmic ointment was provided according to the following formula.

| | |
|---|---|
| Amosulalol hydrochloride | 1.0 g |
| Liquid paraffin | 1.0 g |
| White petrolatum | q. s. |
| | Total 100 g |

Example 9 Ophthalmic Ointment

An ophthalmic ointment was provided according to the following formula.

| | |
|---|---|
| Amosulalol hydrochloride | 0.5 g |
| Liquid paraffin | 1.0 g |
| White petrolatum | q. s. |
| | Total 100 g |

Test Example 1

The effect of topically applied amosulalol hydrochloride on the blood flow of optic nerve head induced by intravitreous injection of ET-1 in pigmented rabbits.

1. Experimental animals

Male Dutch pigmented rabbits weighing about 2 kg were purchased from Fukusaki Rabbit Farmers Association and used after confirmation of absence of ocular abnormality. The rabbits were housed at 23 ±3 °C and 55±10% R.H. and put on a ration of 100 g/day of rabbit chow (Labo R Stock, Nippon Nosan Kogyo K.K.) with free access to tap water.

2. Test Drugs

As the investigational drug, the anti-ocular circulatory disturbance composition containing 0.5% (w/v) of amosulalol hydrochloride as obtained in Example 7 was used. As a control drug, physiological saline solution was used.

3. Methods

(1) Injection of ET-1

In both eyes of rabbits under mydriasis, 10 µl of $10^{-6}$M ET-1 (human, SIGMA) was injected into the center of the vitreous body using a microsyringe (Hamilton, 25 µl) under constant fundus monitoring with a vitrectomy lens (Sun Contact Lens).

(2) Measurement of blood flow

The rabbit was placed in a retainer for rabbits and, after instillation of Mydrin P (Registered Trademark, Santen Pharmaceutical) for mydriasis and observation of the optic nerve head for any abnormality, general anesthesia was introduced by injecting urethane (dissolved in distilled water to 20% concentration), 1 g/kg, subcutaneously in the abdominal region. Under anesthesia which had steadied after 1-2 hours, a dish-shaped indifferent electrode (Biomedical Science, BE-R10) was installed beneath the cranial skin and both eyes were opened by pulling apart sutures passed through the upper and lower eyelids. The bulbar conjunctiva at 6 o'clock was incised and a suture was passed through the inferior rectus muscle and pulled down and fixed. The sclera about 3 mm from the corneo-scleral limbus at 6 o'clock of the eyeball was incised with a 27G needle and a needle indifferent electrode (Biochemical Science, BE-NSP 450-30) was inserted from the incision through the vitreous body into the optic nerve head. The incised part of the

sclera and the different electrode were fixed in position with Aron-Alpha (Registered Trademark, Konishi). After a time lag of about 1 hour, hydrogen gas of 10% concentration was inspirated for about 5 minutes and the distance from the baseline to the apex of the clearance curve traced on a recorder was measured at 12-second intervals. The data were plotted on semi-log paper and the best straight line was drawn through the plots. The half-life ($T_{1/2}$) was determined from the line and the blood flow was calculated by means of Kety's theoretical formula (Journal of Clinical Investigate, 27, 476-483, 1948).

$$\text{Blood flow (ml/min./100 g)} = 69.3/T_{1/2}$$

Measurement of blood flow was made from 30 minutes before administration of ET-1 to 2 hours after administration at 30-minute intervals. The initial baseline value of blood flow was the average of the two steady values found by determination at 15-minute intervals.

(3) Instillation of Drug

Sixty (60) and 30 minutes before administration of ET-1, immediately prior to administration, and 60 minutes after administration, 50 μl of the investigational drug was instilled in one eye and the same volume of physiological saline solution in the contralateral eye.

(4) Results

The initial blood flow values in the respective animals were within the range of 27.7-70.0 ml/min./100 g and the mean value was 47.1 ml/min./100 g. The relative blood flow on optic nerve head of the control eye and the eye treated with the investigational drug (the blood flow values at respective points of time with the initial value being taken as 100%) are shown in Fig. 1 with time (min.) at the time of ET-injection taken as 0 in abscissa and relative blood flow values (%) on optic nerve head as ordinate; each values in mean ± S.E. (number of cases is 6). Black points denote amosulalol ophthalmic solution (Test Drug) and white points denote physiological saline solution (Control). Significant difference from the control (physiological saline solution) *1: $p<0.05$, *2: $p<0.01$ (paired t-test).

In the control eye, a significant decrease in blood flow compared with the initial value was found during the time from 30 to 120 minutes after intravitreous injection of ET-1, with a maximum decrease of 36.3% being found at 90 minutes after ET-1 injection. However, in the eye treated with the investigational drug, no decrease of blood flow was observed at all and, investigational drug significantly inhibited the ET-1-induced decrease in blood flow during the time from 30 to 120 minutes after ET-1 injection.

(5) Conclusion

To this day, most of the reports about the effectiveness of drugs other than amosulalol hydrochloride in the same ocular disorder model as used in Test Example 1 involved intravitreous injection, but actually this intravitreous route of administration cannot be used clinically. Therefore, the above finding that topical amosulalol hydrochloride inhibited the ET-1-induced decrease in blood flow is of great clinical significance. It is, therefore, concluded that amosulalol hydrochloride is a clinically useful drug for ocular circulatory disturbance.

As inferable from the results of Test Example 1, amosulalol hydrochloride inhibits the decrease of blood flow on optic nerve head induced by injection of ET-1 and, therefore, is of value as a therapeutic and prophylactic drug for various diseases caused by ocular circulatory disturbance, such as normal tension glaucoma, pigmentary retinal degeneration, macular regeneration, ischemic optic neuropathy, iridocyclitis, retinal artery occlusion, retinal vein occlusion, diabetic retinopathy, choroidal disease secondary to retinal lesions, and retina-choroidal disease accompanied by systemic disorders.

Also noticeable are descriptions in the above-mentioned WO93/24121 that the acute toxicity of amosulalol hydrochloride with mice ($LD_{50}$ in oral administration and intravenous administration) is very low and that the preparation according to the present invention as shown in Example 5 (0.5% amosulalol hydrochloride ophthalmic solution) and a 0.1% amosulalol hydrochloride ophthalmic solution made in the same manner did not show any topical toxicity in rabbits' eyes such as eye irritation after consecutive instillation for 28 days.

Industrial Utilization

The anti-ocular circulatory disturbance composition according to the present invention has an excellent anti-ocular circulatory disturbance action even by ocular administration and shows little or no side effects such as toxicity. It can thus be used as a therapeutic and prophylactic drug for diseases caused by ocular circulatory disturbance, such as

normal tension glaucoma, pigmentary retinal degeneration, macular regeneration, ischemic optic neuropathy, iridocyclitis, retinal artery occlusion, retinal vein occlusion, diabetic retinopathy, choroidal disease secondary to retinal lesions, and retina-choroidal disease, without a risk for adverse reactions.

## Claims

1. An anti-ocular circulatory disturbance composition comprising 5-[1-hydroxy-2-[2-(2-methoxyphenoxy)ethylamino]ethyl]-2-methylbenzenesulfonamide of the following formula:

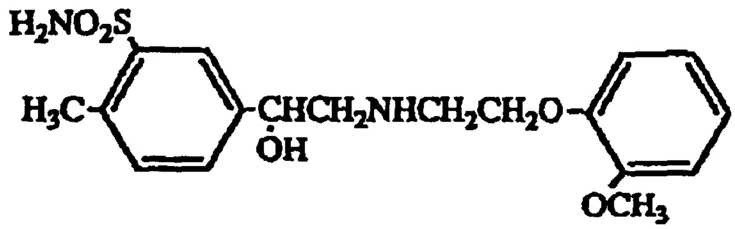

or an acid addition salt thereof as an active ingredient.

2. An anti-ocular circulatory disturbance composition according to Claim 1, the dosage form of which is an ophthalmic solution.

3. An anti-ocular circulatory disturbance composition according to Claim 1, the dosage form of which is an ophthalmic ointment.

4. An anti-ocular circulatory disturbance composition according to Claim 2 or 3 which is a prophylactic and therapeutic drug for a disease caused by circulatory disturbance of the ciliary arterial system.

5. An anti-ocular circulatory disturbance composition according to Claim 4 wherein the disease caused by circulatory disturbance of the ciliary arterial system is a disease selected from the group consisting of normal tension glaucoma, pigmentary retinal degeneration, macular degeneration, ischemic optic neuropathy, and iridocyclitis.

6. An anti-ocular circulatory disturbance composition according to Claim 2 or 3 wherein the ocular circulatory disturbance is a circulatory disturbance of the central retinal arterial system.

7. An anti-ocular circulatory disturbance composition according to Claim 6 wherein the disease caused by circulatory disturbance of the central retinal arterial system is a disease selected from the group consisting of retinal artery occlusion, retinal vein occlusion, diabetic retinopathy, ischemic optic neuropathy, choroidal disease secondary to retinal lesions, and retina-choroidal disease accompanied by systemic disorders.

8. A method of treating a disease caused by circulatory disturbance of the ciliary arterial system, comprising administering to a human subject in need thereof an effective amount in treating the disease of 5-[1-hydroxy-2-[2-(2-methoxyphenoxy)-ethylamino]ethyl]-2-methylbenzenesulfonamide of the following formula:

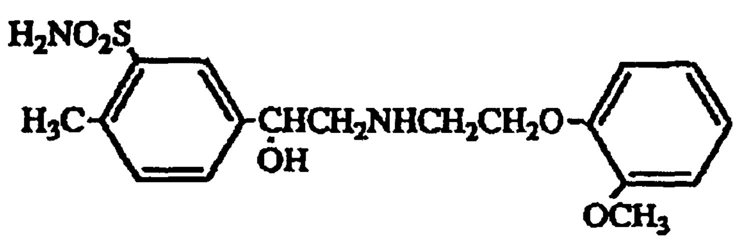

or an acid addition salt thereof as an active ingredient.

**9.** A method according to Claim 8 wherein the disease caused by circulatory disturbance of the ciliary arterial system is a disease selected from the group consisting of normal tension glaucoma, pigmentary retinal degeneration, macular degeneration, ischemic optic neuropathy, and iridocyclitis.

**10.** A method according to Claim 8 wherein the disease caused by circulatory disturbance of the central retinal arterial system is a disease selected from the group consisting of retinal artery occlusion, retinal vein occlusion, diabetic retinopathy, ischemic optic neuropathy, choroidal disease secondary to retinal lesions, and retina-choroidal disease accompanied by systemic disorders.

**11.** The use of 5-[1-hydroxy-2-[2-(2-methoxyphenoxy)ethylamino]ethyl]-2-methylbenzenesulfonamide of the following formula:

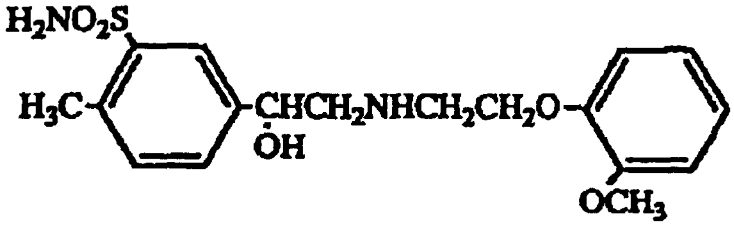

or an acid addition salt thereof in the manufacture of a medicament for the treatment of a disease caused by circulatory disturbance of the ciliary arterial system.

**12.** A use according to Claim 11 wherein the disease caused by circulatory disturbance of the ciliary arterial system is a disease selected from the group consisting of normal tension glaucoma, pigmentary retinal degeneration, macular degeneration, ischemic optic neuropathy, and iridocyclitis.

**13.** A use according to Claim 11 wherein the disease caused by circulatory disturbance of the central retinal arterial system is a disease selected from the group consisting of retinal artery occlusion, retinal vein occlusion, diabetic retinopathy, ischemic optic neuropathy, choroidal disease secondary to retinal lesions, and retina-choroidal disease accompanied by systemic disorders.

**Amended claims under Art. 19.1 PCT**

1. (amended) An anti-ocular circulatory disturbance composition for topical ocular administration comprising 5-[1-hydroxy-2-[2-(2-methoxyphenoxy)ethylamino]ethyl]-2-methylbenzenesulfonamide of the following formula:

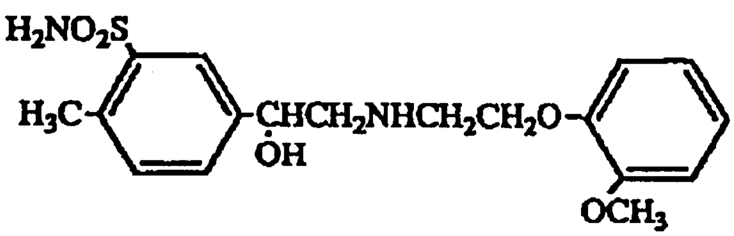

or an acid addition salt thereof as an active ingredient.

2. An anti-ocular circulatory disturbance composition according to Claim 1, the dosage form of which is an ophthalmic solution.

3. An anti-ocular circulatory disturbance composition according to Claim 1, the dosage form of which is an ophthalmic ointment.

4. An anti-ocular circulatory disturbance composition according to Claim 2 or 3 which is a prophylactic and therapeutic drug for a disease caused by circulatory disturbance of the ciliary arterial system.

5. An anti-ocular circulatory disturbance composition according to Claim 4 wherein the disease caused by circulatory disturbance of the ciliary arterial system is a disease selected from the group consisting of normal tension glaucoma, pigmentary retinal degeneration, macular degeneration, ischemic optic neuropathy, and iridocyclitis.

6. An anti-ocular circulatory disturbance composition according to Claim 2 or 3 wherein the ocular circulatory disturbance is a circulatory disturbance of the central retinal arterial system.

7. An anti-ocular circulatory disturbance composition according to Claim 6 wherein the disease caused by circulatory disturbance of the central retinal arterial system is a disease selected from the group consisting of retinal artery occlusion, retinal vein occlusion, diabetic retinopathy, ischemic optic neuropathy, choroidal disease secondary to retinal lesions, and retina-choroidal disease accompanied by systemic disorders.

8. (amended) A method of treating a disease caused by circulatory disturbance of the ciliary arterial system, comprising administering topically to the eyes of a human subject in need thereof an effective amount in treating the disease of 5-[1-hydroxy-2-[2-(2-methoxyphenoxy)ethylamino]ethyl]-2-methylbenzenesulfonamide of the following formula:

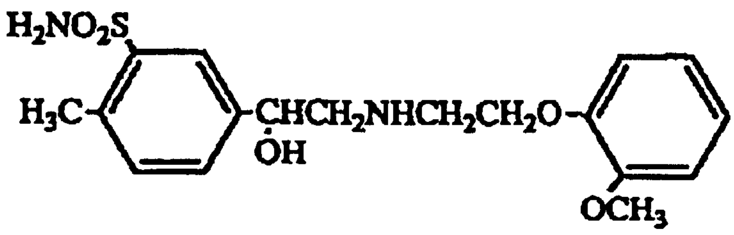

or an acid addition salt thereof as an active ingredient.

9. A method according to Claim 8 wherein the disease caused by circulatory disturbance of the ciliary arterial system is a disease selected from the group consisting of normal tension glaucoma, pigmentary retinal degeneration, macular degeneration, ischemic optic neuropathy, and iridocyclitis.

10. A method according to Claim 8 wherein the disease caused by circulatory disturbance of the central retinal arterial system is a disease selected from the group consisting of retinal artery occlusion, retinal vein occlusion, diabetic retinopathy, ischemic optic neuropathy, choroidal disease secondary to retinal lesions, and retina-choroidal disease accompanied by systemic disorders.

11. (amended) The use of 5-[1-hydroxy-2-[2-(2-methoxyphenoxy)ethylamino]ethyl]-2-methylbenzenesulfonamide of the following formula:

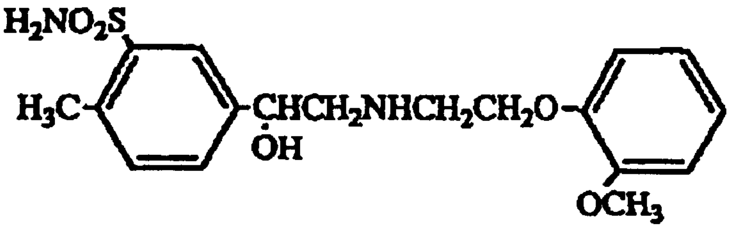

or an acid addition salt thereof in the manufacture of a medicament for topical ocular administration for the treatment of a disease caused by circulatory disturbance of the ciliary arterial system.

12. A use according to Claim 11 wherein the disease caused by circulatory disturbance of the ciliary arterial system is a disease selected from the group consisting of normal tension glaucoma, pigmentary retinal degeneration, macular degeneration, ischemic optic neuropathy, and iridocyclitis.

13. A use according to Claim 11 wherein the disease caused by circulatory disturbance of the central retinal arterial system is a disease selected from the group consisting of retinal artery occlusion, retinal vein occlusion, diabetic retinopathy, ischemic optic neuropathy, choroidal disease secondary to retinal lesions, and retina-choroidal disease accompanied by systemic disorders.

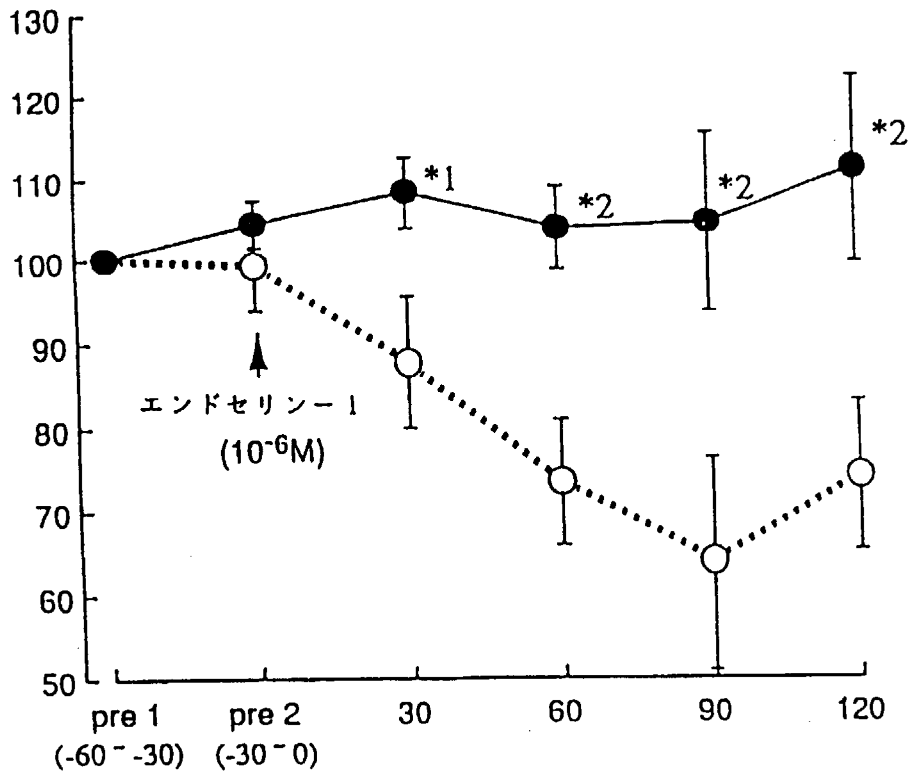
130
120
110
100
90
80
70
60
50
*1
*2
*2
*2
エンドセリン－1
(10-6M)
pre 1
(-60 ~ -30)
pre 2
(-30 ~ 0)
30
60
90
120

## INTERNATIONAL SEARCH REPORT

International application No.
PCT/JP97/02629

**A. CLASSIFICATION OF SUBJECT MATTER**

Int. Cl[6] A61K31/18, A61K9/06, A61K9/08

According to International Patent Classification (IPC) or to both national classification and IPC

**B. FIELDS SEARCHED**

Minimum documentation searched (classification system followed by classification symbols)

Int. Cl[6] A61K31/18, A61K9/06, A61K9/08

Documentation searched other than minimum documentation to the extent that such documents are included in the fields searched

Electronic data base consulted during the international search (name of data base and, where practicable, search terms used)

CA(STN), WRIDS(STN), MEDLINE(STN)

**C. DOCUMENTS CONSIDERED TO BE RELEVANT**

| Category* | Citation of document, with indication, where appropriate, of the relevant passages | Relevant to claim No. |
|---|---|---|
| X | Toru Taniguchi et al., "The influence of amosulalol hydrochloride on rabbit ocular tension and aqueous humor kinetics (in Japanese)", Acta Societatis Ophthalmologicae Japonicae, Vol. 100, No. 4, (1996, April), pages 279 to 283; particularly refer to page 279, abstract; page 279 to page 280, left column, line 18; I. Foreword; page 282, left column, lines 20 to 48 & Database Medline on STN, US National Library of Medicine, (Bethesda, MD, USA), No. 96231699, TANIGUCHI, T., et al., 'Effect of amosulalol hydrochloride on intraocular pressure and aqueous humor dynamics in the rabbit eye,' abstract. | 1-7, 11-13 |
| A | WO, 93/24121, A1 (Senju Pharmaceutical Co., Ltd.), December 9, 1993 (09. 12. 93), Full descriptions & EP, 642789, A1 & CN, 1080849, A | 1-7, 11-13 |

☐ Further documents are listed in the continuation of Box C. ☐ See patent family annex.

* Special categories of cited documents:
"A" document defining the general state of the art which is not considered to be of particular relevance
"E" earlier document but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| October 9, 1997 (09. 10. 97) | October 21, 1997 (21. 10. 97) |
| Name and mailing address of the ISA/<br>Japanese Patent Office<br>Facsimile No. | Authorized officer<br><br>Telephone No. |

Form PCT/ISA/210 (second sheet) (July 1992)

**INTERNATIONAL SEARCH REPORT**

International application No.
PCT/JP97/02629

C (Continuation). DOCUMENTS CONSIDERED TO BE RELEVANT

| Category* | Citation of document, with indication, where appropriate, of the relevant passages | Relevant to claim No. |
|---|---|---|
| A | WO, 94/01096, A1 (Telor Ophthalmic Pharmaceuticals, Inc.), January 20, 1994 (20. 01. 94), Full descriptions & EP, 648118, A1 & JP, 7-508751, A & US, 5488050, A | 1-7, 11-13 |

Form PCT/ISA/210 (continuation of second sheet) (July 1992)